# EUROPEAN PATENT APPLICATION

(11) **EP 4 772 171 A1**
(43) Date of publication of application: **08.07.2026**
(21) Application number: 24862120.3
(22) Date of filing: 09.09.2024
(51) Int. Cl.: A61K 9/10, A61K 9/08, A61K 9/06, A61K 31/496, A61P 25/24, A61P 25/18, A61P 25/14, A61P 25/28

(54) **GEL FOR INJECTION**

(30) Priority: 08.09.2023 CN 202311156607
(71) Applicant: Bostal Drug Delivery Co., Ltd., Guangzhou, Guangdong 510530 (CN); Bostal LLC, Edison, New Jersey 08837 (US)
(72) Inventor: WU, Shaoxian, Guangzhou, Guangdong 510530 (CN); YUE, Zhanguo, Guangzhou, Guangdong 510530 (CN); LI, Wei, Guangzhou, Guangdong 510530 (CN); LIU, Rong, Edison New Jersey 08837 (US)
(74) Representative: Lavoix
(86) International application number: PCT/CN2024/117846
(87) International publication number: WO 2025/051282

(57) **Abstract**

A gel dosage form of brexpiprazole for injection, comprising a solution containing brexpiprazole or a pharmaceutically acceptable salt thereof, and a solution containing at least one biocompatible biodegradable sustained-release material, wherein the two solutions are mixed before being administered to a patient in need thereof, and are mixed to form a clear solution, and the solution containing brexpiprazole or a pharmaceutically acceptable salt thereof can be formed by mixing a powder of brexpiprazole or the pharmaceutically acceptable salt thereof with a solvent.

## Description

### CROSS-REFERENCE TO RELATED APPLICATION

The present application claims priority to the Chinese Patent Application No. 202311156607.4, filed with the China National Intellectual Property Administration on September 8, 2023, entitled "GEL", and the entire contents of which are incorporated herein by reference.

### TECHNICAL FIELD

The present disclosure generally relates to the pharmaceutical field, and specifically to a gel dosage form for injection.

### BACKGROUND

Schizophrenia is a severe mental disorder that clinically manifests as a syndrome with diverse symptoms, involving impairments in perception, thought, emotion, behavior, and other aspects, as well as disorganization of mental activity. The course of the disease is protracted, with recurrent episodes or exacerbations, and some patients ultimately experience deterioration and mental disability.

Brexpiprazole, as an agonist of 5-HT1A receptor and dopamine D2 receptor and an antagonist of 5-HT2A receptor, is clinically used for the treatment of schizophrenia, major depressive disorder, and agitation associated with dementia due to Alzheimer's disease. It demonstrates superior efficacy and tolerability, reducing adverse reactions such as akathisia, restlessness, or insomnia in patients. At present, for the treatment of patients with mental illness, the poor compliance with oral administration of brexpiprazole leads to a high relapse rate of the disease. Therefore, it is necessary to develop other dosage forms of brexpiprazole.

### SUMMARY OF THE INVENTION

The object of the present application is to develop a gel dosage form of brexpiprazole for injection administration. However, studies have found that gel dosage form of brexpiprazole is prone to crystallization during preparation or storage, especially the gel dosage form of brexpiprazole with high concentrations. How to reduce crystallization and enable gel dosage form of brexpiprazole to be administered in the form of a solution is an urgent problem to be solved in the development of gel dosage form of brexpiprazole. Furthermore, the present application can reduce crystallization in high concentration products that prone to crystallization using conventional preparation methods, resulting in better physical stability and clinical safety of the products.

In one aspect, the present disclosure relates to a gel dosage form of brexpiprazole comprising brexpiprazole or a pharmaceutically acceptable salt thereof, at least one biocompatible and biodegradable sustained-release material, and at least one solvent, wherein the concentration of the brexpiprazole or a pharmaceutically acceptable salt thereof is not less than 80 mg/mL.

In another aspect, the present disclosure relates to a gel dosage form of brexpiprazole comprising a solution containing brexpiprazole or a pharmaceutically acceptable salt thereof, and a solution containing at least one biocompatible and biodegradable sustained-release material, wherein the two solutions are mixed before being administered to a patient in need thereof, and the mixed solution is a clear solution.

Among them, the solution containing brexpiprazole or a pharmaceutically acceptable salt thereof can be formed by mixing a powder of brexpiprazole or the pharmaceutically acceptable salt thereof with a solvent.

In yet another aspect, the present disclosure relates to a method for use as an adjunctive therapy to the treatment of major depressive disorder in adults, comprising administering an effective amount of gel dosage form of brexpiprazole to a patient in need thereof, wherein the gel dosage form of brexpiprazole comprises brexpiprazole or a pharmaceutically acceptable salt thereof, at least one biocompatible and biodegradable sustained-release material, and at least one solvent, and wherein the concentration of the brexpiprazole or a pharmaceutically acceptable salt thereof is not less than 80 mg/mL.

In a further aspect, the present disclosure relates to a method for treating schizophrenia, comprising administering an effective amount of gel dosage form of brexpiprazole to a patient in need thereof, wherein the gel dosage form of brexpiprazole comprises brexpiprazole or a pharmaceutically acceptable salt thereof, at least one biocompatible and biodegradable sustained-release material, and at least one solvent, and wherein the concentration of the brexpiprazole or a pharmaceutically acceptable salt thereof is not less than 80 mg/mL.

In yet another aspect, the present disclosure relates to a method for treating agitation associated with dementia due to Alzheimer's disease, comprising administering an effective amount of gel dosage form of brexpiprazole to a patient in need thereof, wherein the gel dosage form of brexpiprazole comprises brexpiprazole or a pharmaceutically acceptable salt thereof, at least one biocompatible and biodegradable sustained-release material, and at least one solvent, and wherein the concentration of the brexpiprazole or a pharmaceutically acceptable salt thereof is not less than 80 mg/mL.

The present disclosure also provides a method for preparing the gel dosage form of brexpiprazole, wherein a solution containing brexpiprazole or a pharmaceutically acceptable salt thereof and a solution containing at least one biocompatible and biodegradable sustained-release material are first prepared. Prior to use, the solution containing brexpiprazole or a pharmaceutically acceptable salt thereof is mixed with the solution containing at least one biocompatible and biodegradable sustained-release material to form the gel dosage form.

The present disclosure also provides a method for preparing the gel dosage form of brexpiprazole, wherein a solution containing at least one biocompatible and biodegradable sustained-release material is first prepared. Prior to use, the brexpiprazole or a pharmaceutically acceptable salt thereof is dissolved in a solvent to obtain a solution containing brexpiprazole or a pharmaceutically acceptable salt thereof and then mixed with the solution containing at least one biocompatible and biodegradable sustained-release material to form the gel dosage form.

In some embodiments, the content of the brexpiprazole or a pharmaceutically acceptable salt thereof is not less than 28 mg/g. Optionally, the content of the brexpiprazole or a pharmaceutically acceptable salt thereof ranges from 28 mg/g to 120 mg/g.

In some embodiments, based on the total mass of the brexpiprazole or a pharmaceutically acceptable salt thereof, the sustained-release material and the solvent, when the content of the brexpiprazole or a pharmaceutically acceptable salt thereof is less than 80 mg/g, the sum of three times the content of the brexpiprazole or a pharmaceutically acceptable salt thereof and the content of the sustained-release material in the gel dosage form of brexpiprazole is not more than 500 mg/g, preferably not more than 450 mg/g.

In some embodiments, when the content of the brexpiprazole or a pharmaceutically acceptable salt thereof is less than 80 mg/g, based on the total mass of the brexpiprazole or a pharmaceutically acceptable salt thereof, the sustained-release material and the solvent, the sum of three times the content of the brexpiprazole or a pharmaceutically acceptable salt thereof and the content of the sustained-release material in the gel dosage form of brexpiprazole is not more than 430 mg/g, preferably not more than 415 mg/g, and more preferably not more than 395 mg/g.

In some embodiments, based on the total mass of the brexpiprazole or a pharmaceutically acceptable salt thereof, the sustained-release material and the solvent, the sum of three times the content of the brexpiprazole or a pharmaceutically acceptable salt thereof and the content of the sustained-release material is not less than 322 mg/g.

In some embodiments, the content of the sustained-release material is not less than 80 mg/g. Optionally, the content of the sustained-release material ranges from 80 mg/g to 300 mg/g.

In some embodiments, when the content of the brexpiprazole or a pharmaceutically acceptable salt thereof is not less than 80 mg/g, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material in the gel dosage form of brexpiprazole is not more than 320mg/g, preferably not more than 270mg/g, and more preferably not more than 235mg/g.

In some embodiments, when the content of the brexpiprazole or a pharmaceutically acceptable salt thereof is not less than 80 mg/g, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material in the gel dosage form of brexpiprazole is not more than 320mg/g, preferably not more than 270mg/g, and more preferably not more than 235mg/g.

The gel dosage form mentioned therein is a solution at room temperature. When the content of the brexpiprazole or a pharmaceutically acceptable salt thereof is not less than 80 mg/g, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material in the gel dosage form of brexpiprazole is not more than 230mg/g, preferably not more than 210mg/g, and more preferably not more than 190mg/g.

In some embodiments, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material is not less than 180mg/g.

The present disclosure also provides use of gel dosage form of brexpiprazole in the manufacture of a medicine for use as an adjunctive therapy to the treatment of major depressive disorder in adults.

The present disclosure also provides use of gel dosage form of brexpiprazole in the manufacture of a medicine for treating schizophrenia.

The present disclosure also provides use of gel dosage form of brexpiprazole in the manufacture of a medicine for assisting in the treatment of agitation associated with dementia due to Alzheimer's disease.

The present disclosure relates to a method for use as an adjunctive therapy to the treatment of major depressive disorder in adults, comprising administering an effective amount of gel dosage form of brexpiprazole to a patient in need thereof.

The present disclosure relates to a method for treating schizophrenia, comprising administering an effective amount of gel dosage form of brexpiprazole to a patient in need thereof.

The present disclosure relates to a method for treating agitation associated with dementia due to Alzheimer's disease, comprising administering an effective amount of gel dosage form of brexpiprazole to a patient in need thereof.

### Description

In the following description, certain specific details are included to provide a comprehensive understanding of various disclosed embodiments. However, those skilled in the relevant art will recognize that the embodiments may still be practiced without utilizing one or more of these specific details, and by utilizing other methods, components, materials, and the like.

Unless otherwise required in the present application, throughout the specification, the words "including", "comprising", "containing" and "having" shall be interpreted as open-ended and inclusive in meaning, i.e., "including but not limited to".

As used in the present disclosure and the accompanying specification, unless the context clearly dictates otherwise, singular references without a numerical indicator include plural references.

Throughout the specification, references to "an embodiment", "embodiments", "in another embodiment" or "in certain embodiments" mean that at least one embodiment includes the specific referenced element, structure, or feature described in that embodiment. Thus, the phrases "in an embodiment", "in embodiments", "in another embodiment" or "in certain embodiments" appearing in various places throughout the specification do not necessarily all refer to the same embodiment. Furthermore, specific elements, structures, or features may be combined in any suitable manner in one or more embodiments.

It should be understood that the singular articles "a", "an" and "the" used in the specification of the present disclosure include plural objects unless the context clearly dictates otherwise. Thus, for example, reference to a sustained-release tablet containing "a pharmaceutically acceptable excipient" includes one pharmaceutically acceptable excipient, or two or more pharmaceutically acceptable excipients.

### Definitions

In the present disclosure, the term "brexpiprazole" refers to 7-{4-[4-(1-benzothiophen-4-yl)piperazin-1-yl]butoxy}quinolin-2(1*H*)-one.

In the present disclosure, the term "acceptable salt" refers to an acid addition salt or base addition salt prepared from a pharmaceutically acceptable acid or base; acceptable acid addition salts include, but are not limited to, benzenesulfonic acid, acetic acid, propionic acid, oxalic acid, isobutyric acid, maleic acid, malonic acid, benzoic acid, trans-butenedioic acid, succinic acid, suberic acid, lactic acid, mandelic acid, gluconic acid, phthalic acid, *p*-toluenesulfonic acid, citric acid, salicylic acid, tartaric acid, methanesulfonic acid, isonicotinic acid, acid citrate, oleic acid, tannic acid, pantothenic acid, hydrogen tartrate, ascorbic acid, gentisic acid, fumaric acid, saccharic acid, formic acid, ethanesulfonic acid, amino acids, hydrochloric acid, hydrobromic acid, hydroiodic acid, nitric acid, carbonic acid, phosphoric acid, phosphorous acid, sulfuric acid, etc.; acceptable base addition salts include, but are not limited to, diethanolamine salts, calcium salts, ammonium salts, lithium salts, sodium salts, potassium salts, aluminum salts, magnesium salts, zinc salts, bismuth salts, etc.

In the present disclosure, the term "biocompatible and biodegradable sustained-release material" refers to natural or synthetic biomedical materials that are biocompatible and acceptable, capable of regulating medicine release rates, and continuously degrading under the action of bodily fluids to be absorbed or excreted by the body, ultimately being completely replaced by new tissue.

In the present disclosure, the term "gel dosage form", also known as an injectable gel dosage form, is typically composed mainly of an active pharmaceutical ingredient, a biocompatible solvent, and a biodegradable polymer. After being injected in a liquid state, the gel dosage form undergoes a rapid phase transition at the administration site, transforming from a liquid to a solid state, thereby prolonging medicine retention time at the site of administration. Simultaneously, as the polymer slowly degrades, the encapsulated medicine is released completely within a few weeks to months, achieving the purpose of sustained and controlled release.

In the present disclosure, the term "room temperature" refers to 20°C to 30°C.

In the present application, the term "solution" is also referred to as a single-phase homogeneous mixture, meaning a dispersion system in which one pure substance is homogeneously and stably distributed in another pure substance in molecular or ionic form, without precipitate or deposit visible to the naked eye.

In the present application, the term "clear" means that the gel dosage form contains no precipitate or deposit visible to the naked eye.

In the present application, the term "crystallization" means that the gel dosage form contains precipitate or deposit visible to the naked eye.

In the present application, the term "Incremental Intrusion" refers to the amount of mercury entering the pore space of a sample as pressure increases during porosity testing by mercury intrusion porosimetry. It is a method to measure how the pore space is filled with mercury under applied pressure, providing results on the internal pore structure and distribution of the sample.

In the present application, the term "burst release peak plasma drug concentration" refers to the first peak plasma drug concentration rapidly achieved by the medicine within a short period after administration.

In the present application, the term "second peak plasma drug concentration" refers to a relatively high peak plasma drug concentration achieved again after sustained release of the medicine.

In the present application, the term "AUC₀₋ₜ" refers to the area under the plasma drug concentration-time curve from time zero to time t, where t denotes the sampling time of the last measurable concentration.

In the present application, the term "AUC_{inf}" refers to the area under the plasma drug concentration-time curve from time zero to infinity.

The term "T_{1/2}" refers to the time required for the blood drug concentration to decrease by half.

### DESCRIPTION OF DRAWINGS

Figure 1 shows the effect of the sum of three times the API content and the PLGA content on crystallization behavior when the API content is not less than 80 mg/g.
Figure 2 shows the effect of the sum of API content and PLGA content on crystallization behavior when the API content is not less than 80 mg/g.
Figure 3 shows the effect of the sum of three times the API content and the PLGA content on crystallization behavior when the API content is less than 80 mg/g.
Figure 4 shows the pore size distribution test results of the gel dosage form of brexpiprazole after solidification.
Figure 5a shows the plasma drug concentration-time curve for the pharmacokinetics (PK) of the gel dosage form of brexpiprazole in rats over 0-2 days.
Figure 5b shows the plasma drug concentration-time curve for the pharmacokinetics (PK) of the gel dosage form of brexpiprazole in rats over 0-42 days.

### DETAILED DESCRIPTION

In one aspect, the present disclosure relates to a gel dosage form of brexpiprazole comprising brexpiprazole or a pharmaceutically acceptable salt thereof, at least one biocompatible and biodegradable sustained-release material, and at least one solvent, wherein the concentration of the brexpiprazole or a pharmaceutically acceptable salt thereof is not less than 80 mg/mL.

In certain embodiments, the concentration of brexpiprazole or a pharmaceutically acceptable salt thereof in the gel dosage form of brexpiprazole of the present disclosure is not less than 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL, or 130 mg/mL.

In certain embodiments, the sum of three times the concentration of brexpiprazole or a pharmaceutically acceptable salt thereof and the concentration of the sustained-release material in the gel dosage form of brexpiprazole of the present disclosure is not more than 600 mg/mL, 595 mg/mL, 590 mg/mL, 585 mg/mL, 580 mg/mL, 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL, or 490 mg/mL.

In certain embodiments, exemplary examples of biocompatible and biodegradable sustained-release materials capable of being used in the gel dosage forms of the present disclosure include, but are not limited to, polyamides, polyamines, poly(alkylene oxalates), polyanhydrides, polyamide esters, copoly(ether-ester), polycarbonates, polyorthoesters, polylactides, polyglycolides, polylactic acid, poly(lactic-co-glycolic acid) copolymers, polyorthoesters, polycaprolactones, polyphosphazenes, polysaccharides, protein polymers, derivatives of polysaccharides, soluble derivatives of protein polymers, polypeptides, polyesters, polyorthoesters, as well as copolymers, block copolymers, homopolymers, mixtures, and compositions thereof.

In certain embodiments, exemplary examples of biocompatible and biodegradable sustained-release materials capable of being used to form the microspheres of the present disclosure include, but are not limited to, poly(lactic-co-glycolic acid) copolymers with a molar ratio of lactic acid (lactide) to glycolic acid (glycolide) ranging from approximately 50:50 to approximately 95:5, also known as poly(lactic-co-glycolic acid), abbreviated as PLGA.

In certain embodiments, exemplary examples of at least one solvent capable of being used in the gel dosage form of the present disclosure include, but are not limited to, organic solvents.

In certain embodiments, exemplary examples of organic solvents capable of being used in the present disclosure include, but are not limited to, alcohol solvents, ester solvents, ether solvents, ketone solvents, and amide solvents.

In certain embodiments, exemplary examples of alcohol solvents capable of being used in the present disclosure include, but are not limited to, ethanol, n-propanol, isopropanol, n-butanol, propylene glycol, glycerol, or 1,3-butanediol.

In certain embodiments, exemplary examples of ester solvents capable of being used in the present disclosure include, but are not limited to, ethyl acetate, methyl acetate, ethyl formate, butyl acetate, diethyl malonate, n-butyl acetate, or isobutyl acetate.

In certain embodiments, exemplary examples of ether solvents capable of being used in the present disclosure include, but are not limited to, tetrahydrofuran, methyltetrahydrofuran, diethylene glycol monomethyl ether, or tetrahydrofurfuryl alcohol polyethylene glycol ether.

In certain embodiments, exemplary examples of ketone solvents capable of being used in the present disclosure include, but are not limited to, acetone, methyl ethyl ketone, diethyl ketone, or methyl isobutyl ketone.

In certain embodiments, exemplary examples of amide solvents capable of being used in the present disclosure include, but are not limited to, formamide, dimethylformamide, dimethylacetamide, hexamethylphosphoramide, N-methyl-2-pyrrolidone, N-cyclohexyl-2-pyrrolidone, N-hydroxyethyl-2-pyrrolidone, 2-pyrrolidone, or N-ethyl-2-pyrrolidone.

In a further aspect, the present disclosure relates to a gel dosage form of brexpiprazole comprising a solution containing brexpiprazole or a pharmaceutically acceptable salt thereof, and a solution containing at least one biocompatible and biodegradable sustained-release material, wherein the two solutions are mixed before being administered to a patient in need thereof, and the mixed solution is a clear solution.

In certain embodiments, the solution containing brexpiprazole or a pharmaceutically acceptable salt thereof of the present disclosure can be formed by mixing a powder of brexpiprazole or the pharmaceutically acceptable salt thereof with a solvent.

In certain embodiments, the gel dosage form of brexpiprazole of the present disclosure is a clear solution at room temperature.

In certain embodiments, the concentration of brexpiprazole or a pharmaceutically acceptable salt thereof in the gel dosage form of brexpiprazole of the present disclosure is 80 mg/mL, 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL, or 130 mg/mL.

In certain embodiments, the sum of three times the concentration of brexpiprazole or a pharmaceutically acceptable salt thereof and the concentration of the sustained-release material in the gel dosage form of brexpiprazole of the present disclosure is not more than 600 mg/mL, 595 mg/mL, 590 mg/mL, 585 mg/mL, 580 mg/mL, 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL, or 490 mg/mL.

In yet another aspect, the present disclosure relates to a method for use as an adjunctive therapy to the treatment of major depressive disorder in adults, comprising administering to a patient in need thereof an effective amount of a gel dosage form of brexpiprazole. The gel dosage form comprises brexpiprazole or a pharmaceutically acceptable salt thereof, at least one biocompatible and biodegradable sustained-release material, and at least one solvent, wherein the concentration of brexpiprazole or a pharmaceutically acceptable salt thereof is not less than 80 mg/mL, 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL, or 130 mg/mL.

In certain embodiments, in a gel dosage form capable of being used in the administration methods of the present disclosure, the sum of three times the concentration of brexpiprazole or a pharmaceutically acceptable salt thereof and the concentration of the sustained-release material is not more than 600 mg/mL, 595 mg/mL, 590 mg/mL, 585 mg/mL, 580 mg/mL, 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL, or 490 mg/mL.

In certain embodiments, a gel dosage form capable of being used in the administration methods of the present disclosure comprises a solution containing brexpiprazole or a pharmaceutically acceptable salt thereof and a solution containing at least one biocompatible and biodegradable sustained-release material. The two solutions are mixed before being administered to a patient in need thereof to form a solution.

In a further aspect, the present disclosure relates to a method for treating schizophrenia, comprising administering an effective amount of gel dosage form of brexpiprazole to a patient in need thereof, wherein the gel dosage form of brexpiprazole comprises brexpiprazole or a pharmaceutically acceptable salt thereof, at least one biocompatible and biodegradable sustained-release material, and at least one solvent.

In certain embodiments, in a gel dosage form of brexpiprazole capable of being used in the administration methods of the present disclosure, the concentration of brexpiprazole or a pharmaceutically acceptable salt thereof is not less than 80mg/mL, 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL, or 130 mg/mL.

In certain embodiments, in a gel dosage form of brexpiprazole capable of being used in the administration methods of the present disclosure, the sum of three times the concentration of brexpiprazole or a pharmaceutically acceptable salt thereof and the concentration of the sustained-release material is not more than 600 mg/mL, 595 mg/mL, 590 mg/mL, 585 mg/mL, 580 mg/mL, 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL, or 490 mg/mL.

In certain embodiments, a gel dosage form capable of being used in the administration methods of the present disclosure comprises a solution containing brexpiprazole or a pharmaceutically acceptable salt thereof and a solution containing at least one biocompatible and biodegradable sustained-release material. The two solutions are mixed before being administered to a patient in need thereof to form a solution.

In certain embodiments, a gel dosage form capable of being used in the administration method of the present disclosure is a solution at room temperature.

In yet another aspect, the present disclosure relates to a method for treating agitation associated with dementia due to Alzheimer's disease, comprising administering an effective amount of gel dosage form of brexpiprazole to a patient in need thereof, wherein the gel dosage form of brexpiprazole comprises brexpiprazole or a pharmaceutically acceptable salt thereof, at least one biocompatible and biodegradable sustained-release material, and at least one solvent.

In certain embodiments, in a gel dosage form of brexpiprazole capable of being used in the administration methods of the present disclosure, the concentration of brexpiprazole or a pharmaceutically acceptable salt thereof is not less than 80mg/mL, 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL, or 130 mg/mL.

In certain embodiments, in a gel dosage form of brexpiprazole capable of being used in the administration methods of the present disclosure, the sum of three times the concentration of brexpiprazole or a pharmaceutically acceptable salt thereof and the concentration of the sustained-release material is not more than 600 mg/mL, 595 mg/mL, 590 mg/mL, 585 mg/mL, 580 mg/mL, 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL, or 490 mg/mL.

In certain embodiments, a gel dosage form capable of being used in the administration methods of the present disclosure comprises a solution containing brexpiprazole or a pharmaceutically acceptable salt thereof and a solution containing at least one biocompatible and biodegradable sustained-release material. The two solutions are mixed before being administered to a patient in need thereof to form a solution.

In certain embodiments, a gel dosage form capable of being used in the administration method of the present disclosure is a solution at room temperature.

In certain embodiments, the gel dosage form of the present disclosure provides sustained release of brexpiprazole or a pharmaceutically acceptable salt thereof for at least approximately one month.

In yet another aspect, the present disclosure also provides a method for preparing the gel dosage form of brexpiprazole, wherein a solution containing brexpiprazole or a pharmaceutically acceptable salt thereof and a solution containing at least one biocompatible and biodegradable sustained-release material are first prepared. Prior to use, the solution containing brexpiprazole or a pharmaceutically acceptable salt thereof is mixed with the solution containing at least one biocompatible and biodegradable sustained-release material to form the gel dosage form.

In certain embodiments, a solution containing brexpiprazole or a pharmaceutically acceptable salt thereof can be formed by mixing brexpiprazole or the pharmaceutically acceptable salt thereof with a solvent. A solution containing at least one biocompatible and biodegradable sustained-release material may be prepared by mixing the sustained-release material with a solvent.

In yet another aspect, the present disclosure also provides a method for preparing the gel dosage form of brexpiprazole, wherein a solution containing at least one biocompatible and biodegradable sustained-release material is first prepared. Prior to use, the brexpiprazole or a pharmaceutically acceptable salt thereof is dissolved in a solvent to obtain a solution containing brexpiprazole or a pharmaceutically acceptable salt thereof and then mixed with the solution containing at least one biocompatible and biodegradable sustained-release material to form the gel dosage form.

Containers for preparing or mixing the two solutions may be conventional vessels such as vials, screw-neck glass bottles, blue cap bottles, polytetrafluoroethylene (PTFE) bottles, polypropylene bottles, etc.

In certain embodiments, a solution containing at least one biocompatible and biodegradable sustained-release material may be prepared by mixing the sustained-release material with a solvent.

In some embodiments, when the content of the brexpiprazole or a pharmaceutically acceptable salt thereof is less than 80 mg/g (e.g., not less than 28 mg/g and less than 80 mg/g), based on the total mass of the brexpiprazole or a pharmaceutically acceptable salt thereof, the sustained-release material and the solvent, the sum of three times the content of the brexpiprazole or a pharmaceutically acceptable salt thereof and the content of the sustained-release material in the gel dosage form of brexpiprazole is not more than 500mg/g. For example, the sum of three times the content of the brexpiprazole or a pharmaceutically acceptable salt thereof and the content of the sustained-release material in the gel dosage form of brexpiprazole may be 500 mg/g, 480 mg/g, 460 mg/g, 450 mg/g, 440 mg/g, 420 mg/g, 405 mg/g, 390 mg/g, 380 mg/g, 370 mg/g, 360 mg/g, 350 mg/g, 340 mg/g, 330 mg/g, 320 mg/g, 310 mg/g, 300 mg/g, 280 mg/g, 260 mg/g, 250 mg/g, 240 mg/g, 230 mg/g, 220 mg/g, 200 mg/g, 150 mg/g, 100 mg/g, 50 mg/g, or within a range formed by any two of the aforementioned values. For example, the content of the brexpiprazole or a pharmaceutically acceptable salt thereof may be 30 mg/g, 32 mg/g, 34 mg/g, 46 mg/g, 48 mg/g, 50 mg/g, 52 mg/g, 55 mg/g, 58 mg/g, 60 mg/g, 64 mg/g, 66 mg/g, 65 mg/g, 68 mg/g, 70 mg/g, 72 mg/g, 74 mg/g, 76 mg/g, 78 mg/g, 79 mg/g, or within a range formed by any two of the aforementioned values.

In some embodiments, based on the total mass of the brexpiprazole or a pharmaceutically acceptable salt thereof, the sustained-release material and the solvent, the sum of three times the content of the brexpiprazole or a pharmaceutically acceptable salt thereof and the content of the sustained-release material is not less than 322 mg/g. For example, the sum of three times the content of the brexpiprazole or a pharmaceutically acceptable salt thereof and the content of the sustained-release material in the gel dosage form of brexpiprazole may be 500 mg/g, 480 mg/g, 460 mg/g, 450 mg/g, 440 mg/g, 420 mg/g, 405 mg/g, 390 mg/g, 380 mg/g, 370 mg/g, 360 mg/g, 350 mg/g, 340 mg/g, 330 mg/g, or within a range formed by any two of the aforementioned values.

In some embodiments, for example, the sum of three times the content of the brexpiprazole or a pharmaceutically acceptable salt thereof and the content of the sustained-release material in the gel dosage form of brexpiprazole may be 358.5 mg/g, 363.6 mg/g, 366.7 mg/g, 371.4 mg/g, 387.8 mg/g, 389.8 mg/g, 398.4 mg/g, 400 mg/g, 405.8 mg/g, 410.7 mg/g, 422.2 mg/g, 428.6 mg/g, 440 mg/g, 440.9 mg/g, 444.4 mg/g, 454.5 mg/g, 478.3 mg/g, or within a range formed by any two of the aforementioned values.

In some embodiments, the content of the brexpiprazole or a pharmaceutically acceptable salt thereof is not less than 28 mg/g. Optionally, the content of the brexpiprazole or a pharmaceutically acceptable salt thereof ranges from 28 mg/g to 120 mg/g. For example, the content of the brexpiprazole or a pharmaceutically acceptable salt thereof may be 28.6 mg/g, 45.5 mg/g, 50 mg/g, 54.5 mg/g, 55.6 mg/g, 56.6 mg/g, 61.2 mg/g, 66.7 mg/g, 80 mg/g, 87 mg/g, 90.9 mg/g, 93.8 mg/g, 95.2 mg/g, 101.7 mg/g, 107.1 mg/g, or within a range formed by any two of the aforementioned values.

In some embodiments, the content of the sustained-release material is not less than 80 mg/g. Optionally, the content of the sustained-release material ranges from 80 mg/g to 300 mg/g. For example, the content of the sustained-release material may be 84.7 mg/g, 89.3 mg/g, 117.2 mg/g, 142.9 mg/g, 144.9 mg/g, 166.7 mg/g, 181.8 mg/g, 188.7 mg/g, 200 mg/g, 204.1 mg/g, 217.4 mg/g, 222.2 mg/g, 227.3 mg/g, 250 mg/g, 277.3 mg/g, 277.8 mg/g, 285.7 mg/g, or within a range formed by any two of the aforementioned values.

In some embodiments, based on the total mass of the brexpiprazole or a pharmaceutically acceptable salt thereof, the sustained-release material and the solvent, when the content of the brexpiprazole or a pharmaceutically acceptable salt thereof is not less than 80 mg/g, the sum of the content of the brexpiprazole or a pharmaceutically acceptable salt thereof and the content of the sustained-release material in the gel dosage form of brexpiprazole is not more than 320 mg/g. For example, the sum of the content of brexpiprazole or a pharmaceutically acceptable salt thereof and the content of the sustained-release material in the gel dosage form of brexpiprazole may be 320 mg/g, 310 mg/g, 300 mg/g, 280 mg/g, 260 mg/g, 250 mg/g, 240 mg/g, 230 mg/g, 220 mg/g, 200 mg/g, 150 mg/g, 100 mg/g, 50 mg/g, or within a range formed by any two of the aforementioned values. For example, the content of the brexpiprazole or a pharmaceutically acceptable salt thereof is 80 mg/g, 82 mg/g, 84 mg/g, 86 mg/g, 88 mg/g, 90 mg/g, 92 mg/g, 95 mg/g, 98 mg/g, 110 mg/g, 114 mg/g, 116 mg/g, 115 mg/g, 118 mg/g, 120 mg/g, or within a range formed by any two of the aforementioned values.

In some embodiments, for example, when the content of the brexpiprazole or a pharmaceutically acceptable salt thereof is not less than 80 mg/g, the sum of the content of the brexpiprazole or a pharmaceutically acceptable salt thereof and the content of the sustained-release material in the gel dosage form of brexpiprazole may be 333.3 mg/g, 331.8 mg/g, 288.9 mg/g, 300.0 mg/g, 265.3 mg/g, 314.3 mg/g, 272.7 mg/g, 216.9 mg/g, 245.3 mg/g, or within a range formed by any two of the aforementioned values.

In some embodiments, when the content of the brexpiprazole or a pharmaceutically acceptable salt thereof is not less than 80 mg/g, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material is not less than 180 mg/g. For example, the sum of the content of the brexpiprazole or a pharmaceutically acceptable salt thereof and the content of the sustained-release material in the gel dosage form of brexpiprazole may be 320 mg/g, 310 mg/g, 300 mg/g, 280 mg/g, 260 mg/g, 250 mg/g, 240 mg/g, 230 mg/g, 220 mg/g, 200 mg/g, 180 mg/g, or within a range formed by any two of the aforementioned values.

In the following section, the present disclosure will be explained in detail through the examples below to facilitate a better understanding of its various aspects and advantages. However, it should be understood that the following examples are non-limiting and are provided solely to illustrate certain embodiments of the present disclosure.

### Example

The reagents and equipment used in the examples of the present disclosure are conventional and commercially available.

### Examples 1 to 18

Examples 1 to 18 provide a series of gel dosage forms of brexpiprazole and their preparation methods. The formulations of the gel dosage forms of brexpiprazole in each example are listed in Table 1, where API refers to brexpiprazole, PLGA refers to poly(lactic-co-glycolic acid) 50:50, and NMP refers to N-methyl-2-pyrrolidone.

The specific preparation methods for these gel dosage forms are as follows:
1. The total formulation amount of N-methyl-2-pyrrolidone was divided into two portions. One portion of N-methyl-2-pyrrolidone served as the solvent for the API solution, with 430 mg of N-methyl-2-pyrrolidone used to dissolve per 60 mg of brexpiprazole. The remaining portion of N-methyl-2-pyrrolidone was used as the solvent for the polymer solution. After preparing the API solution and the polymer solution separately, the two were mixed via a prefilled syringe to obtain the gel dosage form.
   The preparation processes for the API solution and polymer solution are detailed below. Since residues were inevitably present when the solution was transferred from the preparation container to the prefilled syringe, both the API solution and the polymer solution were prepared at 2 to 15 times the target mass.
2. Preparation of the API solution
   Brexpiprazole was placed into a vial, and N-methyl-2-pyrrolidone (430 mg of N-methyl-2-pyrrolidone per 60 mg of brexpiprazole) was added. The mixture was manually shaken for 1 to 2 minutes until the brexpiprazole was completely dissolved. After complete dissolution of the API, the formulation amount of the brexpiprazole solution was filled into a new male prefilled syringe and set aside for use.
3. Preparation of the polymer solution
   The remaining N-methyl-2-pyrrolidone was used to completely dissolve poly(lactic-co-glycolic acid) to obtain the polymer solution. The formulation amount of the polymer solution was filled into a female prefilled syringe and set aside for use.
4. The sum of the components in the male and female prefilled syringes constituted the total formulation amount.
5. During use, the male and female prefilled syringes were coupled. The mixture was pushed back and forth for 1 minute (approximately 60 to 100 cycles) to ensure uniform mixing of the gel, thereby obtaining the gel dosage form.

For example, the preparation method for the gel dosage form of Example 2 was as follows: brexpiprazole (180 mg, 3 units amount) was placed into a vial, and N-methyl-2-pyrrolidone (430 mg of N-methyl-2-pyrrolidone per 60 mg of brexpiprazole) was added. The mixture was manually shaken for 1 to 2 minutes until the brexpiprazole was completely dissolved. After complete dissolution of the API, the formulation amount of the brexpiprazole solution was filled into a new male prefilled syringe and set aside for use. N-methyl-2-pyrrolidone (250 mg, calculated as the formulation amount of NMP minus the amount of NMP used in the brexpiprazole solution in the male prefilled syringe, then multiplied by 5) was used to completely dissolve poly(lactic-co-glycolic acid) (750 mg, 5 units amount) to obtain a polymer solution. The formulation amount of the polymer solution was filled into a female prefilled syringe and set aside for use. The sum of the components in the male and female prefilled syringes equaled the total formulation amount. During use, the male and female prefilled syringes were coupled. The mixture was pushed back and forth for 1 minute (approximately 60 to 100 cycles) to ensure uniform mixing of the gel, thereby obtaining the gel dosage form.

Among these, the PLGA grade used in Example 5 was 5050 0.10-0.30 dL/g, manufactured by Ashland. The PLGA grade used in Examples 1 to 4 and Examples 6 to 18 were 5050 0.16-0.24 dL/g, manufactured by Evonik.

**Table 1 Formulation Table**

| Example | Formulation dosage | | | API content (mg/g) | PLGA content (mg/g) | API content + PLGA content (mg/g) | API content*3 + PLGA content (mg/g) |
|---|---|---|---|---|---|---|---|
| | API/mg | PLGA/mg | NMP/mg | | | | |
| Example 1 | 60 | 180 | 480 | 83.3 | 250.0 | 333.3 | 500.0 |
| Example 2 | 60 | 150 | 480 | 87.0 | 217.4 | 304.3 | 478.3 |
| Example 3 | 60 | 120 | 480 | 90.9 | 181.8 | 272.7 | 454.5 |
| Example 4 | 38.0 | 190.0 | 456.0 | 55.6 | 277.8 | 333.3 | 444.4 |
| Example 5 | 35.0 | 177.9 | 428.8 | 54.5 | 277.3 | 331.8 | 440.9 |
| Example 6 | 60.0 | 150.0 | 540.0 | 80.0 | 200.0 | 280.0 | 440.0 |
| Example 7 | 60 | 90 | 480 | 95.2 | 142.9 | 238.1 | 428.6 |
| Example 8 | 60 | 200 | 640 | 66.7 | 222.2 | 288.9 | 422.2 |
| Example 9 | 60 | 50 | 450 | 107.1 | 89.3 | 196.4 | 410.7 |
| Example 10 | 75.0 | 125.0 | 662.5 | 87.0 | 144.9 | 231.9 | 405.8 |
| Example 11 | 60 | 300 | 840 | 50.0 | 250.0 | 300.0 | 400.0 |
| Example 12 | 50.0 | 62.5 | 420.8 | 93.8 | 117.2 | 211.0 | 398.4 |
| Example 13 | 60 | 50 | 480 | 101.7 | 84.7 | 186.4 | 389.8 |
| Example 14 | 60 | 200 | 720 | 61.2 | 204.1 | 265.3 | 387.8 |
| Example 15 | 30.0 | 300.0 | 720.0 | 28.6 | 285.7 | 314.3 | 371.4 |
| Example 16 | 30.0 | 150.0 | 480.0 | 45.5 | 227.3 | 272.7 | 363.6 |
| Example 17 | 60 | 120 | 650 | 72.3 | 144.6 | 216.9 | 361.4 |
| Example 18 | 60 | 200 | 800 | 56.6 | 188.7 | 245.3 | 358.5 |

In the table, API content = API mass / (API mass + PLGA mass + NMP mass)*100%; PLGA content = PLGA mass / (API mass + PLGA mass + NMP mass)*100%; API content*3 + PLGA content = 3*API mass / (API mass + PLGA mass + NMP mass)*100% + PLGA mass / (API mass + PLGA mass + NMP mass)*100%.

### Comparative Examples 1 to 11

Comparative Examples 1 to 11 provide a series of gel dosage forms of brexpiprazole and their preparation methods. The formulations of the gel dosage forms of brexpiprazole in each comparative example are listed in Table 2. Among them, the proportions of API, PLGA, and NMP in the formulations of gel dosage forms of brexpiprazole in Comparative Examples 1 to 11 are the same as those in Examples 1 to 10 and Example 12, with the difference being the preparation methods. The preparation methods for the gel dosage forms in this series of Comparative Examples 1 to 11 are as follows:
The formulation amount of N-methyl-2-pyrrolidone was added to a 10 mL vial containing the formulation amount of brexpiprazole. The mixture was manually shaken for 1 to 2 minutes until the brexpiprazole was completely dissolved. Then, the formulation amount of PLGA (poly(lactic-co-glycolic acid)) was added to the vial. After sealing, the vial was placed on a roller mixer (Manufacturer: CRYSTAL, Model: MR-020) and mixed at a speed of 20 RPM to 50 RPM until the PLGA was dissolved or uniformly suspended, yielding the gel dosage form.

Among them, the PLGA grade used in Comparative Example 5 was 5050 0.10-0.30dL/g, manufactured by Ashland. The PLGA grade used in Comparative Examples 1 to 4 and Comparative Examples 6 to 11 were 5050 0.16-0.24dL/g, manufactured by Evonik.

**Table 2 Formulation Table**

| Comparative Example | Formulation dosage | | | API content (mg/g) | PLGA content (mg/g) | API content + PLGA content (mg/g) | API content*3 + PLGA content (mg/g) |
|---|---|---|---|---|---|---|---|
| | API/mg | PLGA/mg | NMP/mg | | | | |
| Comparative Example 1 | 120 | 360 | 960 | 83.3 | 250.0 | 333.3 | 500.0 |
| Comparative Example 2 | 120 | 300 | 960 | 87.0 | 217.4 | 304.3 | 478.3 |
| Comparative Example 3 | 120 | 240 | 960 | 90.9 | 181.8 | 272.7 | 454.5 |
| Comparative Example 4 | 100 | 500 | 1200 | 55.6 | 277.8 | 333.3 | 444.4 |
| Comparative Example 5 | 60 | 305 | 735 | 54.5 | 277.3 | 331.8 | 440.9 |
| Comparative Example 6 | 120 | 300 | 1080 | 80.0 | 200.0 | 280.0 | 440.0 |
| Comparative Example 7 | 120 | 180 | 960 | 95.2 | 142.9 | 238.1 | 428.6 |
| Comparative Example 8 | 60 | 200 | 640 | 66.7 | 222.2 | 288.9 | 422.2 |
| Comparative Example 9 | 120 | 100 | 900 | 107.1 | 89.3 | 196.4 | 410.7 |
| Comparative Example 10 | 120 | 200 | 1060 | 87.0 | 144.9 | 231.9 | 405.8 |
| Comparative Example 11 | 240 | 300 | 2020 | 93.8 | 117.2 | 210.9 | 398.4 |

### Experimental Example 1: Crystallization Experiment

For the sample from Example 1, during the preparation of the polymer solution, poly(lactic-co-glycolic acid) and N-methyl-2-pyrrolidone were mixed in a sealed container and left for 5 days. However, the poly(lactic-co-glycolic acid) remained undissolved, making it impossible to prepare a gel dosage form. Therefore, the crystallization experiment was not conducted.
1. The freshly prepared gel dosage forms from Examples 2 to 18 were placed at room temperature. Their gel states were observed under a clarity tester at the following time points: 0h, 1h, 2h, 4h, 8h, 24h, 48h, 66h, 72h, 96h, 120h, 144h, and longer durations (observed every 24 hours after the 72nd hour). The experimental results are shown in Table 3.

**Table 3 Results of the crystallization experiment for gel dosage forms from each example**

| Example | 0 h | 1 h | 2 h | 4 h | 8 h | 24 h | 48 h | 66 h | 72 h | 96 h | 120 h | 144 h |
|---|---|---|---|---|---|---|---|---|---|---|---|---|
| Example 2 | No crystallization | Massive crystallization | - | - | - | - | - | - | - | - | - | - |
| Example 3 | No crystallization | Massive crystallization | - | - | - | - | - | - | - | - | - | - |
| Example 4 | No crystallization | No crystallization | No crystallization | No crystallization | No crystallization | Slight crystallization observed visually | - | - | - | - | - | - |
| Example 5 | No crystallization | No crystallization | No crystallization | No crystallization | No crystallization | Slight crystallization observed visually | - | - | - | - | - | - |
| Example 6 | No crystallization | Slight crystallization observed visually | Slight crystallization observed visually | Slight crystallization observed visually | Massive crystallization | - | - | - | - | - | - | - |
| Example 7 | No crystallization | No crystallization | No crystallization | No crystallization | Slight crystallization observed visually | Slight crystallization observed visually | Slight crystallization observed visually | Massive crystallization | | | | |
| Example 8 | No crystallization | No crystallization | No crystallization | No crystallization | No crystallization | No crystallization | Slight crystallization observed visually | - | | | | |
| Example 9 | No crystallization | No crystallization | No crystallization | No crystallization | No crystallization | No crystallization | No crystallization | No crystallization | No crystallization | Slight crystallization observed visually | - | - |
| Example 10 | No crystallization | No crystallization | No crystallization | No crystallization | No crystallization | No crystallization | No crystallization | Slight crystallization observe d visually | - | - | - | - |
| Example 11 | No cry stalliza tion | No cry stallizat ion | No cryst allization | No cry stalliza tion | No cry stalliza tion | No cry stalliza tion | No cry stalliza tion | No c rysta llizat ion | No cry stalliza tion | Sligh t cry stalli zatio n ob serve d vis ually | - | - |
| Example 12 | No crystallization | No crystallization | No crystallization | No crystallization | No crystallization | No crystallization | No crystallization | No crystallization | Slight crystall ization obser ved vi sually | - | - | - |
| Example 13 | No crystallization | No crystallization | No crystallization | No crystallization | No crystallization | No crystallization | No c crystallization | No crystallization | No crystallization | No crystallization | No crystallization | No crystallization |
| Example 14 | No cry stalliza tion | No cry stallizat ion | No cryst allization | No cry stalliza tion | No cry stalliza tion | No cry stalliza tion | No cry stalliza tion | No c rysta llizat ion | No cry stalliza tion | No c rysta llizat ion | No cry stalliza tion | No cr ystalli zation |
| Example 15 | No cry stalliza tion | No cry stallizat ion | No cryst allization | No cry stalliza tion | No cry stalliza tion | No cry stalliza tion | No cry stalliza tion | No c rysta llizat ion | No cry stalliza tion | No c rysta llizat ion | No cry stalliza tion | No cr ystalli zation |
| Example 16 | No cry stalliza tion | No cry stallizat ion | No cryst allization | No cry stalliza tion | No cry stalliza tion | No cry stalliza tion | No cry stalliza tion | No c rysta llizat ion | No cry stalliza tion | No c rysta llizat ion | No cry stalliza tion | No cr ystalli zation |
| Example 17 | No cry stalliza tion | No cry stallizat ion | No cryst allization | No cry stalliza tion | No cry stalliza tion | No cry stalliza tion | No cry stalliza tion | No c rysta llizat ion | No cry stalliza tion | No c rysta llizat ion | No cry stalliza tion | No cr ystalli zation |
| Example 18 | No cry stalliza tion | No cry stallizat ion | No cryst allization | No cry stalliza tion | No cry stalliza tion | No cry stalliza tion | No cry stalliza tion | No c rysta llizat ion | No cry stalliza tion | No c rysta llizat ion | No cry stalliza tion | No cr ystalli zation |

| | | | | | | | | | | | | |
|---|---|---|---|---|---|---|---|---|---|---|---|---|
| Note: The crystallization condition was determined by visually inspecting the properties of the gel dosage form under a clarity tester (Manufacturer: Jingtuo Instrument, Model: YB-IIA). | | | | | | | | | | | | |

2. The freshly prepared gel dosage forms from Comparative Examples 1 to 10 and Comparative Example 12 were placed at room temperature. Their gel states were continuously monitored under the clarity tester from 0 to 1 hour to record the time at which crystallization was first observed. Subsequently, the gel states were observed under the clarity tester at 2h, 4h, 22h, 24h, and 48h, respectively. The experimental results are shown in Table 4.

**Table 4 Crystallization results of gel dosage forms from each Comparative Example**

| Item | 0-1 h | 2 h | 4 h | 22 h | 24 h | 48 h |
|---|---|---|---|---|---|---|
| Comparative Example 1 | Crystallization at 0 h (crystallization occurs when PLGA is not fully dissolved) | Crystalliz ation | Crystal lization | Crystalliz ation | Crystalliz ation | Crystalli zation |
| Comparative Example 2 | Crystallization at 0 h (crystallization occurs when PLGA is not fully dissolved) | Crystalliz ation | Crystal lization | Crystalliz ation | Crystalliz ation | Crystalli zation |
| Comparative Example 3 | Crystallization at 0 h (crystallization occurs when PLGA is not fully dissolved) | Crystalliz ation | Crystal lization | Crystalliz ation | Crystalliz ation | Crystalli zation |
| Comparative Example 4 | Crystallization at 30 min | Crystalliz ation | Crystal lization | Crystalliz ation | Crystalliz ation | Crystalli zation |
| Comparative Example 5 | Crystallization at 30 min | Crystalliz ation | Crystal lization | Crystalliz ation | Crystalliz ation | Crystalli zation |
| Comparative Example 6 | Crystallization at 0 h (crystallization occurs when PLGA is not fully dissolved) | Crystalliz ation | Crystal lization | Crystalliz ation | Crystalliz ation | Crystalli zation |
| Comparative Example 7 | Crystallization at 0 h (crystallization occurs when PLGA is not fully dissolved) | Crystalliz ation | Crystal lization | Crystalliz ation | Crystalliz ation | Crystalli zation |
| Comparative Example 8 | Crystallization at 0 h (crystallization occurs when PLGA is not fully dissolved) | Crystalliz ation | Crystal lization | Crystalliz ation | Crystalliz ation | Crystalli zation |
| Comparative Example 9 | Crystallization at 0 h (crystallization occurs when PLGA is not fully dissolved) | Crystalliz ation | Crystal lization | Crystalliz ation | Crystalliz ation | Crystalli zation |
| Comparative Example 10 | Crystallization at 0 h (crystallization occurs when PLGA is not fully dissolved) | Crystalliz ation | Crystal lization | Crystalliz ation | Crystalliz ation | Crystalli zation |
| Comparative Example 11 | Crystallization at 5 min | Crystalliz ation | Crystal lization | Crystalliz ation | Crystalliz ation | Crystalli zation |

| | | | | | | |
|---|---|---|---|---|---|---|
| Note: The crystallization condition was determined by visually inspecting the properties of the gel dosage forms under a clarity tester (Manufacturer: Jingtuo Instrument, Model: YB-IIA). | | | | | | |

From the crystallization experiment results presented in Tables 3 and 4, it can be observed that, compared to the comparative examples, except for Example 1, the gel dosage forms obtained in the various Examples of the present application significantly mitigate the issue of easy crystallization of gel dosage form of brexpiprazole during the preparation or storage. This demonstrates that the method of the present application is beneficial for preparing a stable gel dosage form in solution form.

To investigate the crystallization behavior of the gel dosage form of brexpiprazole and further understand the cause of crystallization in Example 1, data from each example were analyzed. It was found that the crystallization of the gel dosage form of brexpiprazole was not correlated with the API content. For example, when comparing Example 6 with Example 13, Example 6 has an API content of 80 mg/g, while Example 13 has an API content of 101.7 mg/g. The crystallization in Example 7 first appeared at 1 hour, which was significantly earlier than the time point of 144 hours at which no crystallization was observed in Example 13. Other examples include the comparisons between Example 2 and Example 10, Example 14 and Example 4, Example 14 and Example 5, and Example 14 and Example 11, among others.

Moreover, when the API content is less than 80 mg/g, the crystallization behavior (e.g., crystallization time) is also not correlated with the sum of API content and PLGA content. For example, comparing Example 8 with Example 15, the sum of API content and PLGA content in Example 8 is 288.9 mg/g, while in Example 15, it is 314.3 mg/g. The crystallization in Example 8 first occurred at 48 hours, which was significantly earlier than the time point of 144 hours at which no crystallization was observed in Example 15.

The inventor unexpectedly discovered that when the API content is less than 80 mg/g, crystallization behavior (such as crystallization time) correlates with the sum of three times the API content and the PLGA content. The lower the sum of three times the API content and the PLGA content, the longer the gel dosage form remains in a solution state and the later the crystallization occurs. By controlling the sum of three times the API content and the PLGA content, not only can a solution-like gel dosage form be prepared, enabling the gel dosage form of brexpiprazole to be injected in a solution form, but also the crystallization can be further reduced, prolonging the solution state duration, thus enhancing the product's physical stability and clinical safety, and making it more convenient to use.

When the sum of three times the API content and the PLGA content exceeds 430 mg/g but is less than or equal to 450 mg/g, the crystallization behavior is further improved, and the gel dosage form maintains a solution state for over 8 hours at room temperature. For example, in Examples 4 and 5, where the sum of three times the API content and the PLGA content is between 440.9 mg/g and 444.4 mg/g, crystallization occurs after the gel dosage form is placed at room temperature for 24 hours (maintaining a solution state for at least 8 hours). When the sum of three times the API content and the PLGA content exceeds 415 mg/g but is less than or equal to 430mg/g, the crystallization behavior is further improved, and the gel dosage form maintains a solution state for over 24 hours at room temperature. For example, in Example 8, where the sum of three times the API content and the PLGA content is 422.2 mg/g, crystallization occurs after the gel dosage form is placed at room temperature for 48 hours (maintaining a solution state for at least 24 hours). When the sum of three times the API content and the PLGA content exceeds 395mg/g but is less than or equal to 415 mg/g, the crystallization behavior is further improved. For example, in Example 11, where the sum of three times the API content and the PLGA content is 400.0 mg/g, crystallization occurs after the gel dosage form is placed at room temperature for 96 hours (maintaining a solution state for at least 72 hours). When the sum of three times the API content and the PLGA content is less than or equal to 395 mg/g, the crystallization behavior is further improved, and the gel dosage form maintains a solution state for over 144 hours at room temperature. For example, in Examples 14 to 18, where the sum of three times the API content and the PLGA content ranges from 358.5 mg/g to 387.8 mg/g, the gel dosage form remains in a solution state after being placed at room temperature for 144 hours.

The inventors unexpectedly discovered that when the API content is not less than 80 mg/g, the crystallization behavior (such as crystallization time) correlates with the sum of the API content and the PLGA content. The lower the sum of API content and PLGA content, the longer the gel dosage form remains in a solution state and the later the crystallization occurs. By controlling the sum of the API content and the PLGA content, not only can a solution-like gel dosage form be prepared, enabling the gel dosage form of brexpiprazole to be injected in a solution form, but also the crystallization can be further reduced, prolonging the solution state duration, and enhancing the product's physical stability and clinical safety, making it more convenient to use.

When the sum of API content and PLGA content exceeds 270 mg/g but is less than or equal to 320 mg/g, the method of the present application can produce a solution-like gel dosage form. However, crystallization occurs after being placed for a period of time. For example, in Examples 2, 3, and 6, where the sum of the API content and the PLGA content ranges from 272.7 mg/g to 304.3 mg/g, crystallization occurs after the gel dosage form is placed at room temperature for 1 hour. When the sum of the API content and the PLGA content exceeds 235mg/g but is less than or equal to 270 mg/g, the crystallization behavior is further improved. For example, in Example 7, where the sum of the API content and the PLGA content is 238.1 mg/g, crystallization occurs after the gel dosage form is placed at room temperature for 8 hours (maintaining a solution state for at least 4 hours). When the sum of the API content and the PLGA content exceeds 230 mg/g but is less than or equal to 235 mg/g, the crystallization behavior is further improved. For example, in Example 10, where the sum of the API content and the PLGA content is 231.9 mg/g, crystallization occurs after the gel dosage form is placed at room temperature for 66 hours (maintaining a solution state for at least 48 hours). When the sum of the API content and the PLGA content exceeds 210 mg/g but is less than or equal to 230 mg/g, the crystallization behavior is further improved. For example, in Example 12, where the sum of the API content and the PLGA content is 210.9 mg/g, crystallization occurs after the gel dosage form is placed at room temperature for 72 hours (maintaining a solution state for at least 66 hours). When the sum of the API content and the PLGA content exceeds 190 mg/g but is less than or equal to 210 mg/g, the crystallization behavior is further improved. For example, in Example 9, where the sum of the API content and the PLGA content is 196.4 mg/g, crystallization occurs after the gel dosage form is placed at room temperature for 96 hours (maintaining a solution state for at least 72 hours). When the sum of the API content and the PLGA content is less than or equal to 190 mg/g, the gel dosage form remains in a solution state for over 144 hours at room temperature. For example, in Example 13, the sum of the API content and the PLGA content is 186.4 mg/g.

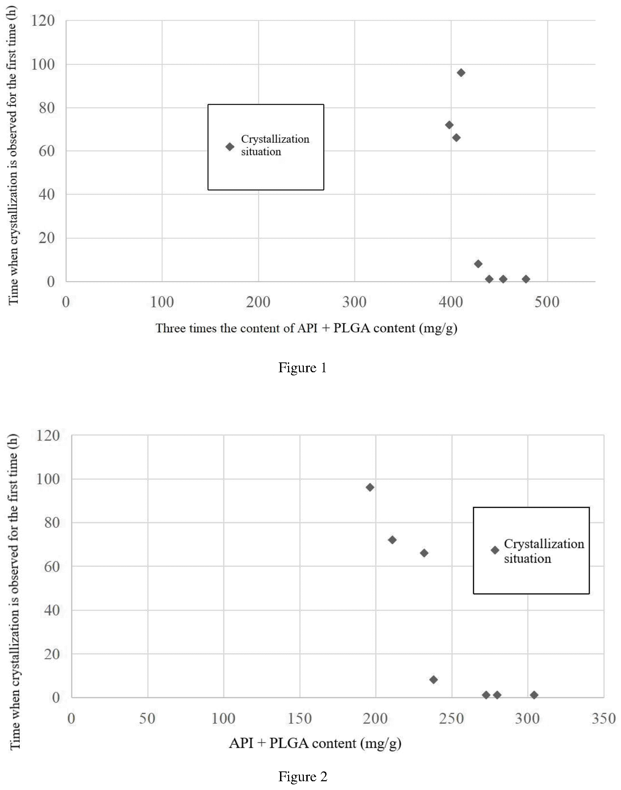

When the API content is not less than 80 mg/g, a relationship diagram illustrating the effect of the sum of three times the API content and the PLGA content on crystallization behavior is plotted (Figure 1), with the sum of three times the API content and the PLGA content (in mg/g) as the x-axis and the time (in hours) at which crystallization is first observed as the y-axis. As shown in Figure 1, when the API content is not less than 80 mg/g, as the sum of three times the API content and the PLGA content decreases, some gel dosage forms maintain their solution state for longer periods when placed at room temperature, while others do so for shorter periods.

When the API content is not less than 80 mg/g, a relationship diagram illustrating the effect of the sum of API content and PLGA content on crystallization behavior is plotted (Figure 2), with the sum of API content and PLGA content (in mg/g) as the x-axis and the time (in hours) at which crystallization is first observed as the y-axis. As shown in Figure 2, when the API content is not less than 80 mg/g, the duration for which the gel dosage form remains in a solution state at room temperature significantly increases as the sum of the API content and the PLGA content decreases.

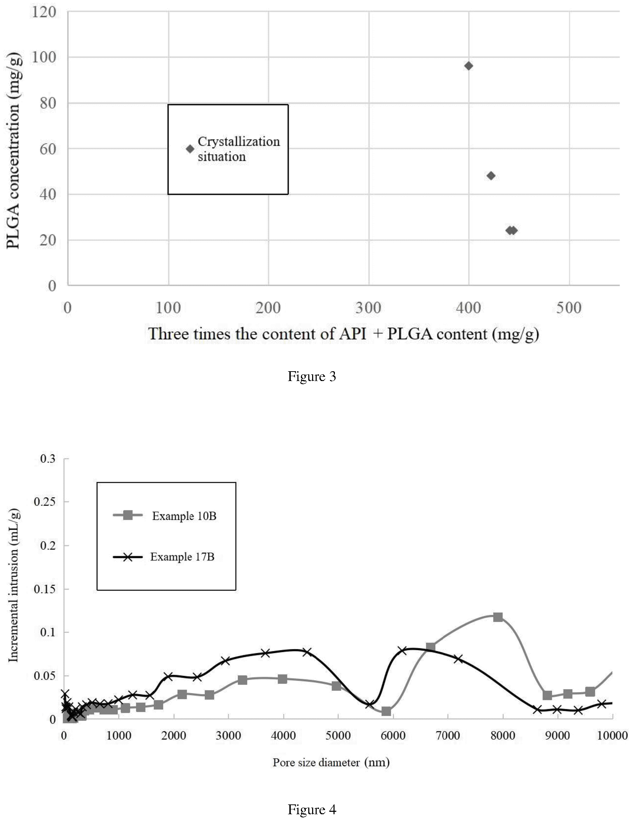

When the API content is less than 80 mg/g, a relationship diagram illustrating the effect of three times the API content and the PLGA content on crystallization behavior is plotted (Figure 3), with the sum of three times the API content and the PLGA content (in mg/g) as the x-axis and the time (in hours) at which crystallization is first observed as the y-axis. As shown in Figure 3, in summary, when the API content is less than 80 mg/g, the duration for which the gel dosage form remains in a solution state at room temperature significantly increases as the sum of three times the API content and the PLGA content decreases.

### Experimental Example 2: Pore Size Distribution Test

The gel dosage form of the present application would solidify after injection to form a solid state. This section determined the pore size distribution inside the solid material formed after the gel dosage form was injected and solidified.
a. Experimental Methods and Instruments:
   Preparation of gel dosage form samples for Example 10B and Example 17B

The contents of API, PLGA, and NMP in the gel dosage form sample of Example 10B are the same as those of the three substances in the formulation of Example 10, respectively. The preparation methods for the gel dosage form samples of Example 10B and Example 17B are as follows:
1) The total formulation amount of N-methyl-2-pyrrolidone was divided into two portions. One portion was used to dissolve the API, i.e., to prepare the API solution. 430 mg of N-methyl-2-pyrrolidone was used to dissolve per 60 mg of brexpiprazole. The remaining N-methyl-2-pyrrolidone was used to prepare the polymer solution. Finally, the API solution and the polymer solution were mixed via a prefilled syringe to obtain the gel dosage form.
2) The preparation processes for the API solution and polymer solution are detailed below. Since residues were inevitably present when the solution was transferred from the preparation container to the prefilled syringe, both the API solution and the polymer solution were prepared at 2 to 10 times the target amount.
3) Preparation of the API solution
   Brexpiprazole sterilized by 25 kGy gamma irradiation was placed into a vial, and N-methyl-2-pyrrolidone sterilized by 25 kGy gamma irradiation (430 mg of N-methyl-2-pyrrolidone per 60 mg of brexpiprazole) was added. The mixture was manually shaken for 1 to 2 minutes until the brexpiprazole was completely dissolved. After complete dissolution of the API, the formulation amount of the brexpiprazole solution was filled into a new male prefilled syringe and set aside for use.
4) Preparation of the polymer solution
   The remaining amount of N-methyl-2-pyrrolidone was used to completely dissolve poly(lactic-co-glycolic acid) to obtain the polymer solution. After sterilization by 25 kGy gamma irradiation, the formulation amount of the polymer solution was filled into a female prefilled syringe and set aside for use.
5) The sum of the components in the male and female prefilled syringes constituted the total formulation amount.
6) During use, the male and female prefilled syringes were coupled. The mixture was pushed back and forth for 1 minute (approximately 60 to 100 cycles) to ensure uniform mixing of the gel, thereby obtaining the gel dosage form.

Specifically, the detailed formulation for the gel dosage form sample in Example 10B was as follows: API 60 mg, PLGA 100 mg, and NMP 530 mg. The preparation method and the amount of each component were as follows:
1) 240 mg (4 times the formulation amount) of brexpiprazole, which had been sterilized by 25 kGy gamma irradiation, was placed into a vial, and N-methyl-2-pyrrolidone (430 mg of N-methyl-2-pyrrolidone per 60 mg of brexpiprazole), also sterilized by 25 kGy gamma irradiation, was then added. The mixture was manually shaken for 1 to 2 minutes until the brexpiprazole was completely dissolved. After complete dissolution of the API, the formulation amount of the brexpiprazole solution was filled into a new male prefilled syringe and set aside for use.
2) 400 mg of N-methyl-2-pyrrolidone was used to completely dissolve 400 mg (4 times the formulation amount) of poly(lactic-co-glycolic acid). After sterilization by 25 kGy gamma irradiation, the polymer solution was obtained. The formulation amount of the polymer solution was filled into a female prefilled syringe and set aside for use.
3) During use, the male and female prefilled syringes are coupled. The mixture was pushed back and forth for 1 minute (approximately 60 to 100 cycles) to ensure uniform mixing of the gel, thereby obtaining the gel dosage form.

The formulation of the gel dosage form sample in Example 17B was the same as that in Example 10, and the preparation method essentially followed that provided for Example 10B. The only difference was that the quantities of each component during the preparation of the polymer solution and the brexpiprazole solution were adjusted accordingly based on the formulation of Example 17B.

### b. Subcutaneous Injection in Rats

Male SD rats weighing approximately 200 g to 400 g were selected and randomly divided into groups. All rats were housed under the environment of 20°C to 25°C and 40% to 70% relative humidity (RH), fed with standard feed, and allowed free access to water and food. The two groups of test rats were subcutaneously injected with the aforementioned two groups of gel dosage form of brexpiprazole, respectively. Each rat received one injection each on the left and right sides of the neck-shoulder region. Based on the mass of brexpiprazole, each injection corresponded to a dose of 15 mg (i.e., 15 mg per injection on each side).

### c. Material Collection

At 48 hours after administration, the rats were sacrificed and the injection sites were dissected. Complete solidified gel dosage form samples were taken out, and those intact in shape and free of tissue adhesion were selected for subsequent freeze-drying.

### d. Freeze-Drying

The extracted gel dosage form samples were placed in 10 ml borosilicate vials for freeze-drying. The freeze-drying equipment and parameters are listed in Table 5.

**Table 5 Freeze-Drying**

| Freeze dryer | | | | | | | | |
|---|---|---|---|---|---|---|---|---|
| Manufacturer | | SP scientific | | | | | | |
| Model | | Advantage Plus-EL-85 | | | | | | |

| Freeze-drying parameters | | | | | | | | |
|---|---|---|---|---|---|---|---|---|
| Pre-freezing | | | | | | | | |
| Temperature | -40°C | | Cooling time | | 60min | Holding time | | 240min |

| Primary drying | | | | | | | | |
|---|---|---|---|---|---|---|---|---|
| Temperature | -10°C | | Heating Time | 60min | Holding time | 1250min | Vacuum degree | 20Pa |

| Secondary drying | | | | | | | | |
|---|---|---|---|---|---|---|---|---|
| Temperature | 0°C | | Heating Time | 30min | Holding time | 1250min | Vacuum degree | 20Pa |
| Temperature | 25°C | | Heating Time | 30min | Holding time | 1250min | Vacuum degree | 20Pa |

### e. After freeze-drying, the samples were used for porosity testing.

Porosity testing instrument information: Mercury porosimeter, Micromeritics, Autopore 9620.

Porosity testing experimental procedure: The sample to be tested was placed into a penetrometer, which was then inserted into the instrument for mercury injection. The results provide the pore volume distribution for pores with diameters ranging from 360 µm to 0.003 µm.

Calculation method of Incremental Intrusion (mL/g): The Incremental Intrusion (mL/g) values (i.e., incremental pore volume) corresponding to the pore size diameter range of 6000 nm to 9000 nm were selected and summed to obtain the result.

### f. Experimental Results:

The pore size distribution test results are shown in Table 6 and Figure 4.

**Table 6 Pore size distribution test results**

| Example/Comparative Example | API content + PLGA content (mg/g) | API content*3+ PLGA content (mg/g) | Incremental intrusion for the 6000-9000 nm pore size distribution range within the solidified gel dosage form |
|---|---|---|---|
| Example 10B | 231.9 | 405.8 | 0.1759 |
| Example 17B | 216.9 | 361.4 | 0.1693 |

### Experimental Example 3: Pharmacokinetic Study in Rats

### Experimental method:

a. Preparation of Gel dosage form Sample in Example 10C:
   The specific formulation of the gel dosage form sample in Example 10C was as follows: API 60 mg, PLGA 100 mg, and NMP 530 mg. The contents of API, PLGA, and NMP in Example 10C were identical to those in the formulation of Example 10, respectively. Moreover, the gel dosage form of brexpiprazole was prepared using the method provided in Example 10B.
b. Experimental Procedure:
   Healthy male SD rats (purchased from Zhuhai Bes Test Bio-Tech Co., Ltd.) weighing approximately 200g to 300 g were selected. There were 3 rats per formulation. All rats were housed under the environment of 20 °C to 25 °C and 40% to 70% relative humidity, fed with standard feed, and allowed free access to water and food. A single dose of the Example 10C gel dosage form sample described above was administered via subcutaneous injection into the lateral buttocks of each test rat. Based on the mass of brexpiprazole, the dosing level of the gel dosage form of brexpiprazole was 18 mg/kg.
   Orbital blood collection time points in rats: blood samples were collected at 0.5 hours, 2 hours, 4 hours, 8 hours, 24 hours, 2 days, 3 days, 5 days, 8 days, 11 days, 14 days, 17 days, 21 days, 24 days, 28 days, 31 days, 35 days, and 39 days after administration.
c. Instruments and Testing Procedure:
   The concentration of brexpiprazole in rat plasma was determined using High-Performance Liquid Chromatography coupled with Triple Quadrupole Mass Spectrometry (HPLC-MS/MS). The linear range was 0.05 ng/mL to 150 ng/mL. A 50 µL rat plasma sample was processed using a protein precipitation method with aripiprazole as the internal standard. Subsequently, the responses for brexpiprazole and its internal standard (aripiprazole) were detected under electrospray (+) ion source conditions to compare the pharmacokinetic profile differences among various formulation preparations.
d. Written Description of PK Curve Plotting:
   The obtained PK data were used to plot PK curves in an EXCEL spreadsheet, with the x-axis representing time and the y-axis representing cumulative plasma drug concentration.

### Experimental results:

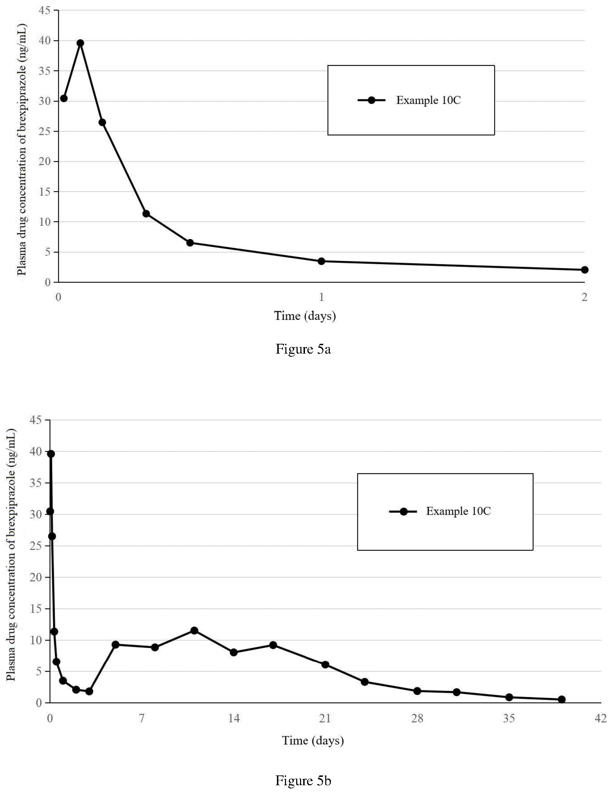

The PK results of gel dosage form in rats are presented in Table 7 and Figures 5a and 5b.

**Table 7 PK Parameters Table**

| | Burst Release Peak | Second Peak |
|---|---|---|
| Time to Peak | 2 hours | 11 days |
| Plasma Drug Concentration (ng/mL) | 39.56 | 11.48 |
| AUC₀₋ₜ (day*ng/mL) | 204.67 | |
| AUC_{inf} (day*ng/mL) | 210.61 | |
| T_{1/2} (day) | 5.0 | |

| | | |
|---|---|---|
| Note: t = 39 days | | |

As can be seen from Figures 5a and 5b, the gel dosage form of the present application exhibits a favorable sustained-release effect.

In the present disclosure, relational terms such as "first" and "second" are used merely to distinguish one entity or operation from another entity or operation, and do not necessarily require or imply any such actual relationship or order between these entities or operations.

It can be understood from the foregoing that although specific embodiments of the present disclosure have been described for illustrative purposes, various modifications or improvements can be made by those skilled in the art without departing from the spirit and scope of the present disclosure. All such modifications or improvements should fall within the scope of the appended specification of the present disclosure.

## Claims

1. A gel dosage form of brexpiprazole, comprising a solution containing brexpiprazole or a pharmaceutically acceptable salt thereof, and a solution containing at least one biocompatible and biodegradable sustained-release material, wherein the two solutions are mixed before being administered to a patient in need thereof to form a clear solution; and
wherein the solution containing brexpiprazole or a pharmaceutically acceptable salt thereof can be formed by mixing a powder of brexpiprazole or a pharmaceutically acceptable salt thereof with a solvent.

2. The gel dosage form according to claim 1, comprising at least one biocompatible and biodegradable sustained-release material and at least one solvent, wherein the concentration of the brexpiprazole or a pharmaceutically acceptable salt thereof is not less than 80 mg/mL.

3. The gel dosage form according to claim 1 or 2, wherein the sum of three times the concentration of the brexpiprazole or a pharmaceutically acceptable salt thereof and the concentration of the sustained-release material is not more than 600 mg/mL.

4. A method for use as an adjunctive therapy to the treatment of major depressive disorder in adults, comprising administering an effective amount of the gel dosage form according to any one of claims 1 to 3 to a patient in need thereof.

5. A method for treating schizophrenia, comprising administering an effective amount of the gel dosage form according to any one of claims 1 to 3 to a patient in need thereof.

6. A method for treating agitation associated with dementia due to Alzheimer's disease, comprising administering an effective amount of the gel dosage form according to any one of claims 1 to 3 to a patient in need thereof.

7. A method for preparing a gel dosage form of brexpiprazole, comprising first preparing a solution containing brexpiprazole or a pharmaceutically acceptable salt thereof and a solution containing at least one biocompatible and biodegradable sustained-release material, then mixing the solution containing brexpiprazole or a pharmaceutically acceptable salt thereof with the solution containing at least one biocompatible and biodegradable sustained-release material before use to form the gel dosage form.

8. A method for preparing a gel dosage form of brexpiprazole, comprising first preparing a solution containing at least one biocompatible and biodegradable sustained-release material, then dissolving the brexpiprazole or a pharmaceutically acceptable salt thereof in a solvent before use to obtain a solution containing brexpiprazole or a pharmaceutically acceptable salt thereof, and subsequently mixing it with the solution containing at least one biocompatible and biodegradable sustained-release material to form the gel dosage form.

9. The gel dosage form of brexpiprazole according to any one of claims 1 to 3, or the gel dosage form of brexpiprazole prepared by the method according to claim 7 or 8, wherein the content of the brexpiprazole or a pharmaceutically acceptable salt thereof is not less than 28 mg/g, optionally, the content of the brexpiprazole or a pharmaceutically acceptable salt thereof ranges from 28 mg/g to 120 mg/g.

10. The gel dosage form of brexpiprazole according to any one of claims 1 to 3, or the gel dosage form of brexpiprazole prepared by the method according to claim 7 or 8, wherein, when the content of the brexpiprazole or a pharmaceutically acceptable salt thereof is less than 80 mg/g, based on the total mass of the brexpiprazole or a pharmaceutically acceptable salt thereof, the sustained-release material and the solvent, the sum of three times the content of the brexpiprazole or a pharmaceutically acceptable salt thereof and the content of the sustained-release material in the gel dosage form of brexpiprazole is not more than 500 mg/g, preferably not more than 450 mg/g.

11. The gel dosage form of brexpiprazole according to any one of claims 1 to 3, or the gel dosage form of brexpiprazole prepared by the method according to claim 7 or 8, wherein, when the content of the brexpiprazole or a pharmaceutically acceptable salt thereof is less than 80 mg/g, based on the total mass of the brexpiprazole or a pharmaceutically acceptable salt thereof, the sustained-release material and the solvent, the sum of three times the content of the brexpiprazole or a pharmaceutically acceptable salt thereof and the content of the sustained-release material in the gel dosage form of brexpiprazole is not more than 430mg/g, preferably not more than 415mg/g, more preferably not more than 395 mg/g.

12. The gel dosage form of brexpiprazole according to any one of claims 1 to 3, or the gel dosage form of brexpiprazole prepared by the method according to claim 7 or 8, wherein, based on the total mass of the brexpiprazole or a pharmaceutically acceptable salt thereof, the sustained-release material and the solvent, the sum of three times the content of the brexpiprazole or a pharmaceutically acceptable salt thereof and the content of the sustained-release material is not less than 322 mg/g.

13. The gel dosage form of brexpiprazole according to any one of claims 1 to 3, or the gel dosage form of brexpiprazole prepared by the method according to claim 7 or 8, wherein the content of the sustained-release material is not less than 80 mg/g, optionally, the content of the sustained-release material ranges from 80 mg/g to 300 mg/g.

14. The gel dosage form of brexpiprazole according to any one of claims 1 to 3, or the gel dosage form of brexpiprazole prepared by the method according to claim 7 or 8, wherein, when the content of the brexpiprazole or a pharmaceutically acceptable salt thereof is not less than 80 mg/g, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material in the gel dosage form of brexpiprazole is not more than 320mg/g, preferably not more than 270mg/g, more preferably not more than 235mg/g.

15. The gel dosage form of brexpiprazole according to any one of claims 1 to 3, or the gel dosage form of brexpiprazole prepared by the method according to claim 7 or 8, wherein, when the content of the brexpiprazole or a pharmaceutically acceptable salt thereof is not less than 80 mg/g, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material in the gel dosage form of brexpiprazole is not more than 230 mg/g, preferably not more than 210 mg/g, more preferably not more than 190 mg/g.

16. The gel dosage form of brexpiprazole according to any one of claims 1 to 3, or the gel dosage form of brexpiprazole prepared by the method according to claim 7 or 8, wherein, based on the total mass of the brexpiprazole or a pharmaceutically acceptable salt thereof, the sustained-release material and the solvent, the sum of the content of the brexpiprazole or a pharmaceutically acceptable salt thereof and the content of the sustained-release material is not less than 180 mg/g.

17. Use of the gel dosage form of brexpiprazole according to any one of claims 1 to 3, or the gel dosage form of brexpiprazole prepared by the method according to claim 7 or 8, in the manufacture of a medicine for use as an adjunctive therapy to the treatment of major depressive disorder in adults.

18. Use of the gel dosage form of brexpiprazole according to any one of claims 1 to 3, or the gel dosage form of brexpiprazole prepared by the method according to claim 7 or 8, in the manufacture of a medicine for treating schizophrenia.

19. Use of the gel dosage form of brexpiprazole according to any one of claims 1 to 3, or the gel dosage form of brexpiprazole prepared by the method according to claim 7 or 8, in the manufacture of a medicine for treating agitation associated with dementia due to Alzheimer's disease.

20. A method for use as an adjunctive therapy to the treatment of major depressive disorder in adults, comprising administering an effective amount of the gel dosage form of brexpiprazole prepared by the method according to claim 7 or 8 to a patient in need thereof.

21. A method for treating schizophrenia, comprising administering an effective amount of the gel dosage form of brexpiprazole prepared by the method according to claim 7 or 8 to a patient in need thereof.

22. A method for treating agitation associated with dementia due to Alzheimer's disease, comprising administering an effective amount of the gel dosage form of brexpiprazole prepared by the method according to claim 7 or 8 to a patient in need thereof.
